(19) Europäisches Patentamt
European Patent Office
Office européen des brevets

(11) Veröffentlichungsnummer : **0 049 745**
**B1**

(12) # EUROPÄISCHE PATENTSCHRIFT

(45) Veröffentlichungstag der Patentschrift : **28.11.84**

(51) Int. Cl.³ : **C 07 C 103/78**, A 61 K 49/04

(21) Anmeldenummer : **81106183.7**

(22) Anmeldetag : **07.08.81**

(54) **Neue N-Hydroxy-alkylierte Dicarbonsäure-bis-(3,5-dicarbamoyl-2,4,6-trijodanilide), deren Herstellung und diese enthaltende Röntgenkontrastmittel.**

(30) Priorität : **10.10.80 DE 3038853**

(43) Veröffentlichungstag der Anmeldung : **21.04.82 Patentblatt 82/16**

(45) Bekanntmachung des Hinweises auf die Patenterteilung : **28.11.84 Patentblatt 84/48**

(84) Benannte Vertragsstaaten : **AT BE CH DE FR GB IT LI LU NL SE**

(56) Entgegenhaltungen :
**EP-A- 0 011 849**
**EP-A- 0 015 867**
**EP-A- 0 026 281**
**DE-A- 2 628 517**
**DE-A- 2 805 928**
**US-A- 4 001 323**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Pfeiffer, Heinrich, Dr.**
**Pulfrichzeile 9**
**D-1000 Berlin 20 (DE)**
Erfinder : **Speck, Ulrich, Dr.**
**Benediktiner Strasse 50**
**D-1000 Berlin 28 (DE)**

**EP 0 049 745 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue N-Hydroxyalkylierte Dicarbonsäure-bis-(3,5-dicarbamoyl-2,4,6-trijod-anilide) der allgemeinen Formel I

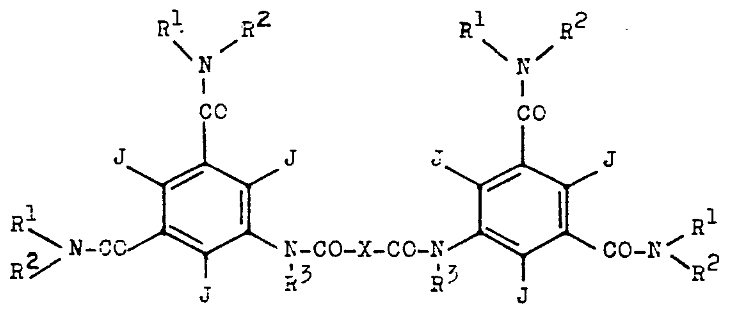

(I)

worin $R^1$ einen niederen gerad- oder verzweigtkettigen Mono- oder Polyhydroxyalkylrest mit 2 bis 8 Kohlenstoffatomen der durch 1 bis 5 Hydroxygruppen substituiert ist, $R^2$ ein Wasserstoffatom, einen mit 1 bis 4 Kohlenstoffatomen oder $R^1$, $R^3$ einen Mono- oder Dihydroxyalkylrest mit 2 bis 3 Kohlenstoffatomen und X eine Direktbindung oder ein gegebenenfalls verzweigtkettiges Alkylen mit 1 bis 6 Kohlenstoffatomen, das durch ein oder mehrere Sauerstoffatome unterbrochen oder durch Hydroxy- oder Alkoxygruppen substituiert sein kann, bedeuten sowie ein Verfahren zu deren Herstellung und neue Röntgenkontrastmittel, die Verbindungen der Formel I als schattengebende Substanz enthalten.

Der Rest $R^1$ enthält als gerad- oder verzweigtkettiger niederer Mono- oder Polyhydroxyalkylrest 2-8 Kohlenstoffatome, vorzugsweise 2-5 Kohlenstoffatome. Geradkettige Reste von $R^1$ bestehen vorzugsweise aus 2-4 Kohlenstoffatomen, verzweigtkettige vorzugsweise aus 3-5 Kohlenstoffatomen. Die Hydroxygruppen im Rest $R^1$ können als primäre oder sekundäre Hydroxygruppen vorliegen. Der Rest $R^1$ kann 1-5 Hydroxygruppen enthalten, bevorzugt sind 1-3 Hydroxygruppen, so daß Verbindungen der allgemeinen Formel I insgesamt 4-40 Hydroxygruppen, vorzugsweise 4-24 Hydroxygruppen, enthalten können. Als Reste $R^1$ seien beispielsweise genannt: 2-Hydroxyäthyl-, 2-Hydroxypropyl-, 3-Hydroxypropyl-, 2,3-Dihydroxy-propyl-, 2,3-Dihydroxybutyl-, 2,4-Dihydroxybutyl-, 3,4-Dihydroxybutyl-, 3-Hydroxy-2-(hydroxymethyl)-propyl-, 2,3-Dihydroxy-1-methyl-propyl-, 2-Hydroxy-3-(hydroxymethyl)-butyl-, 2,3,4-Trihydroxybutyl-, 2,4-Dihydroxy-3-(hydroxymethyl)-butyl-, 3-Hydroxy-2,2-bis-(hydroxymethyl)-propyl-, 4-Hydroxy-3,3-bis-(hydroxymethyl)-butyl-, 4-Hydroxy-2,2-bis-(hydroxymethyl)-butyl-, 2-Hydroxy-1,1-bis-(hydroxymethyl)-äthyl-, 1,3-Dihydroxy-isopropyl-, 2,3-Dihydroxy-1-hydroxy-methyl-propyl-Rest.

Wenn $R^2$ einen niederen Alkylrest bedeutet, sind damit insbesondere geradkettige Reste mit 1-4 Kohlenstoffatomen, bevorzugt 1-2 Kohlenstoffatomen, gemeint, wie beispielsweise Butyl, Propyl, Äthyl und insbesondere Methyl.

Der Rest $R^3$ enthält als niederer Mono- oder Dihydroxyalkylrest 2-3 Kohlenstoffatome und 1 bis 2 Hydroxygruppen, die als primäre oder sekundäre Hydroxygruppen vorliegen können. Besonders bevorzugt ist der Hydroxyäthylrest.

X als gerad- oder verzweigtkettiges Alkylen, das durch ein oder mehrere Sauerstoffatome unterbrochen sein kann, kann 1-6 Kohlenstoffatome enthalten. Bevorzugt ist ein geradkettiges Alkylen mit 1-4 Kohlenstoffatomen, das durch ein oder mehrere, vorzugsweise durch ein, zwei oder auch drei Sauerstoffatome unterbrochen sein kann.

Als Beispiele seien hier genannt: $—CH_2—$, $—(CH_2)_4—$, $—(CH_2—CH_2—O—CH_2—CH_2)—$, $—(CH_2—O—CH_2)_2—$ und $—(CH_2—O—CH_2)_3—$.

Als gerad- oder verzweigtkettige Reste X kommen infrage: $>CHCH_3$, $—[CH_2—C(CH_3)_2—CH_2]—$, $—[CH_2—CH(CH_3)—CH_2—CH_2]—$, $—[\underset{OH}{CH}]_2—$ und ähnliche.

X als gerad- oder verzweigtkettiges Alkylen kann auch durch Alkoxygruppen substituiert sein, wobei Methoxy- oder Äthoxygruppen bevorzugt sind.

Für die Röntgendiagnostik, z. B. der harnableitenden Organe und der angiographisch zu erfassenden Gefäße wurden als Kontrastmittel gut verträgliche Salze von 2,4,6-Trijodbenzoesäuren entwickelt. Diese Substanzen werden vom Organismus bei höherer Dosierung jedoch nicht ohne Nebenwirkungen toleriert, obgleich ihre Toxizität oft gering ist. Eine ausreichende Darstellung des Gefäßsystems, der ableitenden Harnwege, aber auch der cerebrospinalen Höhlen und anderer Systeme erfordert die Anwendung hoher Kontrastmitteldosierungen oder hoch konzentrierter Lösungen. Damit gewinnen der physiko-chemischen Eigenschaften der Kontrastmittel und deren Lösungen stark an Bedeutung, da wesentliche pharmakologische Effekte wie Schmerz, Blutdruckabfall, Gefäßschädigung und viele andere auf diese zurückgeführt werden müssen.

Verglichen mit den Salzen von trijodierten Benzoesäurederivaten haben wasserlösliche, nichtionische Verbindungen eine Reihe von Vorteilen:

Sie besitzen einen geringeren osmotischen Druck und verursachen deshalb bei der angiographischen Anwendung weniger Schmerz und geringere Endothelschädigungen. Weiterhin ist die Harnkonzentration höher und bei subarachnoidaler Injektion kommt es seltener zur Arachnoiditis.

Bei der Myelographie wurde bei Anwendung der nichtionischen Röntgenkontrastmittel eine geringe Neigung zu Krampfzuständen (Epileptogenizität) festgestellt. Es ist allerdings mit diesen Verbindungen wie beispielsweise Metrizamid oder Iopamidol für die Anwendung in der Angiographie und Myelographie nicht gelungen, ausreichend konzentrierte und röntgendichte Lösungen herzustellen, die gegenüber dem Blut oder Liquor nicht hyperton sind.

Gut verträgliche, hexajodierte und nicht-ionische Röntgenkontrastmittel, aus denen hochkonzentrierte und blutisotone wäßrige Lösungen hergestellt werden konnten, wurden erstmals in der DOS 26 28 517 beschrieben. Ähnliche Verbindungen werden inzwischen auch in der DOS 28 059 28 beansprucht.

Obwohl nicht-ionische hexajodierte Röntgenkontrastmittel wegen ihrer günstigen physikochemischen Eigenschaften (besonders starke Hydrophile, niedriger osmotischer Druck) recht gut verträglich sind, sind die mit den bisher beschriebenen Verbindungen erzielten Ergebnisse noch nicht voll zufriedenstellend. Nachteilig ist beispielsweise die hohe Viskosität gerade der gut verträglichen und gut löslichen Verbindungen bzw. die geringe Löslichkeit weniger viskoser Substanzen mit hohem Jodgehalt sowie bestimmte toxische Wirkungen einiger der bisher beschriebenen Verbindungen trotz relativ hoher $DL_{50}$-Werte nach intravenöser Verabreichung.

Es war überraschend, daß eine vergleichsweise geringe chemische Veränderung der in der DOS 26 28 517 beschriebenen Verbindungsklasse zu einer bedeutenden Verbesserung der Verträglichkeit und zu einer Erhöhung der Löslichkeit der erfindungsgemäßen Verbindungen führte, wie aus der Tabelle 1 hervorgeht, in der die erfindungsgemäße Verbindung A (Malonsäure-bis-[3,5-bis-(1,3-dihydroxyisopropyl-carbamoyl)-2,4,6-trijod-N-2-hydroxyäthyl-anilid]) mit Oxalsäure-bis[3,5-bis-(2,3-dihydroxypropyl-N-methyl-carbamoyl)-2,4,6-trijod-anilid] (B) sowie mit Iopamidol (5-d-Hydroxypropionyl-amino]-2,4,6-trijod-isophthalsaüre-bis-[1,3-dihydroxyisopropyl-amid]) und Metrizamid (2-(3-Acetamido-2,4,6-trijod-5-[N-methyl-acetamido]-benzamido)-2-deoxy-D-glucose) in bezug auf osmotischen Druck, Viskosität und Verträglichkeit verglichen werden :

Tabelle I

Osmotischer Druck, Viskosität und Verträglichkeit nicht-ionischer Kontrastmittel unterschiedlicher chemischer Struktur als wäßrige Lösung
(Konzentration : 300 mg/ml)

| Substanz | osmotischer Druck 37°C (at) | Viskosität 37°C (cP) | $DL_{50}$(Ratte) nach i.v. Injektion, Geschwindigkeit: 2 ml/min (g Jod/kg Körpergewicht) |
|---|---|---|---|
| A | 7,2 | 6,8 | mehr als 30 |
| B | 4,3 | 13,7 | 13 |
| Iopamidol | 15,7 | 4,7 | 12 |
| Metrizamid | 12,3 | 6,2 | 12 |

Bemerkenswert ist dabei die außergewöhnlich gute Verträglichkeit der erfindungsgemäßen Verbindungen der Formel I, die dem extrem hohen $DL_{50}$-Wert zu entnehmen ist. Eine Untersuchung der dem Test unterworfenen Tiere ergab eine gegenüber den Vergleichsverbindungen verminderte Nierentoxizität, die bei dieser hohen Dosis ein sehr wesentliches Kriterium ist. Dabei sind die erfindungsgemäß hergestellten Verbindungen in der für die Angiographie gebräuchlichen Konzentration von 300 mg Jod/ml isoton mit dem Blut ; sie sind aufgrund ihrer Viskosität sowohl für eine rasche Injektion als auch für eine Injektion durch feine Nadeln geeignet.

Die erfindungsgemäßen Verbindungen der allgemeinen Formel I sind somit als schattengebende Substanzen hervorragend zur Herstellung von bzw. zur Anwendung in Röntgenkontrastmitteln geeignet. Die neuen Verbindungen besitzen alle Eigenschaften, die von Röntgenkontrastmitteln gefordert werden. Viele sind, obwohl nicht-ionisch, sehr gut wasserlöslich. Die neuen Verbindungen stellen hervorragend verträgliche Röntgenkontrastmittel dar, die in der Angiographie, Urographie, Myelographie, Lymphographie und zur Darstellung verschiedener Körperhöhlen und zu anderen radiologischen Untersuchungen geeignet sind.

Aufgrund ihres schwachen und neutralen Geschmackes eignen sich einige der Verbindungen hervorragend für die orale Applikation und zum Einbringen in die Lunge.

Der den gebräuchlichen Kontrastmitteln anhaftende bittere und Übelkeit auslösende Geschmack ist als schwerwiegender Nachteil insbesondere in der Gastrographie und Bronchographie anzusehen.

Die Erfindung betrifft somit auch neue Röntgenkontrastmittel auf Basis von Verbindungen der allgemeinen Formel I. Die Herstellung der neuen Röntgenkontrastmittel auf Basis der erfindungsgemäßen Verbindungen der allgemeinen Formel I erfolgt in an sich bekannter Weise, z. B. dadurch, daß man die schattengebende Substanz mit den in der Galenik üblichen Zusätzen, z. B. Stabilisatoren wie Natriumedetat, Calcium-di-natriumedetat, physiologisch verträglichen Puffern, Natriumchlorid u. ä., in eine für die intravenöse Applikation geeignete Form bringt. Die Konzentration der neuen Röntgenkontrastmittel im wäßrigen Medium richtet sich ganz nach der röntgendiagnostischen Methode. Die bevorzugten Konzentrationen und Dosierungen der neuen Verbindungen bewegen sich in den Bereichen von 50-500 mg J/ml für die Konzentration und 5-500 ml für die Dosierung. Besonders bevorzugt sind Konzentrationen zwischen 100-400 mg J/ml.

Die Erfindung betrifft ferner ein Verfahren zur Herstellung von Verbindungen der allgemeinen Formel I, das dadurch gekennzeichnet ist, daß man in an sich bekannter Weise ein Dicarbonsäure-bis-(3,5-dicarbamoyl-2,4,6-trijod-anilid) der allgemeinen Formel II

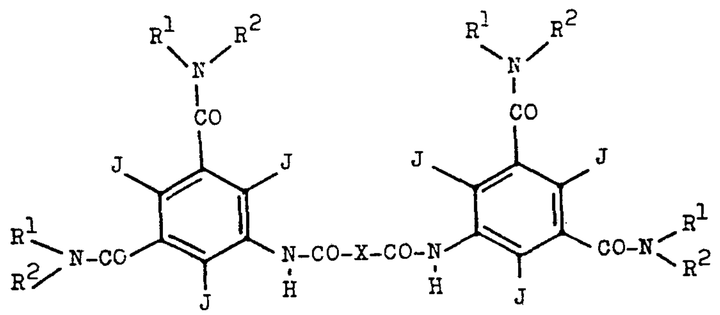

(II)

worin $R^1$, $R^2$ und X die obengenannte Bedeutung besitzt und im Molekül vorhandene freie Hydroxylgruppen in geschützter Form vorliegen können mit einer Verbindung der allgemeinen Formel III

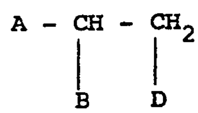

(III)

worin

A ein Wasserstoffatom oder eine $CH_2OH$-Gruppe darstellt und B und D entweder gemeinsam das Sauerstoffatom eines Oxidoringes bedeuten oder

B eine Hydroxygruppe und

D ein Chlor- oder Bromatom oder eine Sulfat- oder Alkylsulftagruppe darstellen,

N-hydroxy-alkyliert und gewünschtenfalls anschließend geschützte Hydroxylgruppen in Freiheit setzt.

Die erfindungsgemäße N-Alkylierung der acylierten Anilidgruppen erfolgt nach dem Fachmann bekannten Methoden. So kann man beispielsweise die Verbindungen der Formel II in einem geeigneten Lösungsmittel wie Methanol, Äthanol oder 1,2-Propandiol in Gegenwart von Alkalialkoholat oder Alkalihydrid mit der Verbindung der Formel III, beispielsweise mit Chloräthanol, Äthylenoxid, Chlorpropandiol-(2,3) oder Glycidol (2,3-Oxido-propanol) bei einer Temperatur von Raumtemperatur bis 80 °C, vorzugsweise von 20 °C bis 50 °C umsetzen.

Eine weitere Möglichkeit der Alkylierung besteht darin, die Verbindung II mit intermediär geschützen Hydroxygruppen in die Reaktion einzusetzen. Dies erfolgt nach üblichen Methoden durch Einführung von leicht wieder abspaltbaren Gruppen, beispielsweise durch Verätherung (z. B. Einführung des Triphenylmethylrestes).

Der Hydroxylgruppenschutz kann auch durch Ketalisierung oder Acetalisierung, z. B. mittels Acetaldehyd, Aceton, 2,2-Dimethoxypropan oder Dihydropyran, erreicht werden.

Die spätere Abspaltung der intermediär eingeführten Schutzgruppen unter Freisetzung der letztlich gewünschten Hydroxylgruppen erfolgt ebenfalls nach Methoden, die dem Fachmann allgemein geläufig sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführt werden. Acylschutzgruppen können beispielsweise durch alkalische und Acetal-, Ketal- oder Ätherschutzgruppen durch saure Hydrolyse abgespalten werden.

Die Ausgangsverbindungen der allgemeinen Formel II sind in der Deutschen Offenlegung 26 28 517, in der französischen Patentschrift 77 19 234 oder in der belgischen Patentschrift 856 039 beschrieben oder können nach an sich bekannten Verfahren aus einem Tetracarbonsäuretetrachlorid der allgemeinen Formel IV

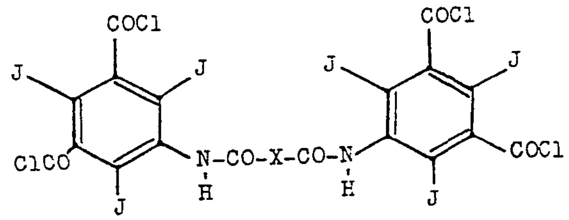

(IV)

das in der Deutschen Offenlegungsschrift 26 28 517 beschrieben ist und worin X die obengenannte Bedeutung besitzt, durch Umsetzung mit einem Amin

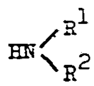

mit $R^1$ und $R^2$ in der oben angegebenen Bedeutung, hergestellt werden, wobei die in $R^1$ und $R^2$ gegebenenfalls vorhandenen freien Hydroxylgruppen in geschützter Form vorliegen können.

Soweit noch nicht beschrieben, lassen sich die Tetracarbonsäuretetrachloride der allgemeinen Formel IV herstellen, indem man in an sich bekannter Weise 5-amino-2,4,6-Trijodisophthalsäuredichlorid der Formel V

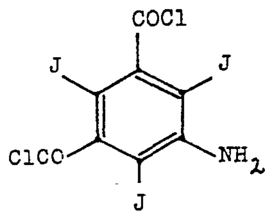

(V)

mit einem Dicarbonsäurechlorid der allgemeinen Formel VI

ClCO—X'—COCl (VI)

worin X' die obengenannte Bedeutung für X hat und zusätzlich gegebenenfalls vorhandene freie Hydroxygruppen in geschützter Form vorliegen können, umsetzt.

Als Schutzgruppen kommen die üblicherweise verwendeten infrage, darüberhinaus auch die 1,2-Oxidogruppe. Diese Umsetzung soll anhand der Herstellungsvorschrift für Malonsäure-bis-[3,5-bis-(chlorcarbonyl)-2,4,6-trijod-anilid] näher erläutert werden :

Die heiße Lösung von 298 g (0,5 Mol) 5-Amino-2,4,6-trijod-isophthalsäure-dichlorid in 650 ml Dioxan wird tropfenweise bei 90 °C Innentemperatur mit 29 ml (0,3 Mol) Malonsäuredichlorid versetzt und unter Rückfluß 2 Stunden gerührt, wobei ein dicker Niederschlag ausfällt. Nach Rühren über Nacht bei Raumtemperatur wird der Niederschlag abfiltriert, mit Dioxan gewaschen und bei 50 °C im Vakuum getrocknet. Man erhält 298 g (79,8 % der Theorie) Malonsäure-bis-[3,5-bis-(chlorcarbonyl)-2,4,6-trijod-anilid], (Dioxangehalt 15,8 %) ; Fp. : 310 °C (Zersetzung).

Die so hergestellten Tetracarbonsäuretetrachloride der allgemeinen Formel IV setzt man nun um mit einem Amin

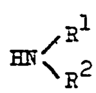

mit $R^1$ und $R^2$ in der oben angegebenen Bedeutung, wobei in $R^1$ und $R^2$ vorhandene freie Hydroxygruppen in geschützter Form vorliegen können und erhält so die Verbindungen der allgemeinen Formel II.

Beispiel 1

Malonsäure-bis-[3,5-bis-(2,3-dihydroxy-propyl-N-methyl-carbamoyl)-2,4,6-trijod-N-2-hydroxyäthyl-anilid]

In eine Lösung von 150 g (100 mMol) Malonsäure-bis-[3,5-bis-(chlorcarbonyl)-2,4,6-trijod-anilid] (Dioxangehalt berücksichtigt) in 250 ml Dimethylacetamid wird bei 50-60 °C unter Rühren eine Lösung von 52,5 g (500 mMol) N-Methyl-amino-propandiol-(2,3) in 150 ml Dimethylacetamid innerhalb 15 Minuten zugetropft, wobei die Temperatur bis auf 59 °C steigen darf. In weiteren 10 Minuten werden 118,8 ml (0,5 Mol) Tributylamin zugegeben. Danach rührt man weitere 4 Stunden bei 50 °C und über Nacht bei

Raumtemperatur. Danach wird das Reaktionsgemisch mit 18,3 ml konzentrierter Salzsäure bis zur sauren Reaktion versetzt und die Lösung in 2 l Methylenchlorid eingetropft. Nach einer stunde Rühren wird vom Niederschlag dekantiert, der Niederschlag wiederum eine Stunde mit 2 l Methylenchlorid gerührt und erneut dekantiert, worauf der Niederschlag bei 50 °C im Vakuum getrocknet wird. Das Rohprodukt (190 g) wird in 1,5 l Wasser gelöst, filtriert, auf eine Säule mit Ca. 2 l Kationenaustauscher IR 120 gegeben und mit Wasser eluiert. Das Eluat wird im Vakuum eingeengt, in 1,5 l Wasser gelöst, auf eine Säule mit ca. 1,5 l Anionenaustauscher IRA 410 gegeben und mit Wasser eluiert. Dieses Eluat wird 30 Minuten mit 15 g Altiv-Kohle gerührt, abgesaugt und im Vakuum eingeengt. Der Rückstand wird bei 50 °C im Vakuum getrocknet. Man erhält 102 g Malonsäure-bis-[3,5-bis-(2,3-dihydroxypropyl-N-methyl-carbamoyl)-2,4,6-trijod-anilid] (66 % der Theorie) ; Fp. : 238-240 °C (Zersetzung).

b) Hydroxyäthylierung :

Unter Erwärmen auf 50 °C werden 3,3 g Natrium (144 mMol) in 60 ml Methanol zum Methylat umgesetzt und schließlich 130 ml 1,2 Propylenglykol zugesetzt. Diese Lösung wird mit 69,9 g (45 mMol) Malonsäure-bis-[3,5-bis-(2,3-dihydroxy-propyl-N-methyl-carbamoyl)-2,4,6-trijod-anilid] und anschließend zur rascheren Lösung mit 300 ml Methanol versetzt. Nach 15 Minuten bei 50 °C ist die Lösung klar. Das Methanol wird dann im Vakuum abdestilliert. Man tropft nun bei ca. 50 °C unter kräftigem Rühren 9 ml (135 mMol) Chloräthanol zu und rührt 5 Stunden bei 50 °C, Nach Abkühlen auf Raumtemperatur wird die schwach gelbliche Trüche Lösung tropfenweise in 2 l aceton eingerührt. Nach einer Stunde Rühren wird der Niederschlag abgesaugt, 30 Minuten mit 500 ml Aceton ausgerührt, wiederum abgesaugt und bei 50 °C im Vakuum getrocknet. Das Rohprodukt wird in 500 ml Wasser gelöst und die Lösung auf eine Säule mit ca. 500 ml Kationenaustauscher IR 120 gegeben. Nach Elution mit Wasser wurde im Vakuum auf 400 ml eingeengt und auf eine Säule mit 500 ml Anionenaustauscher (IRA 410) gegeben. Nach Elution mit wasser wurde das Eluat 30 Minuten mit 4 g Kohle gerührt, abgesaugt und im Vakuum eingeengt. Man erhält 59,3 g Titelverbindung (80 % der Theorie) ; Fp. : 247-249 °C (Zersetzung).

### Beispiel 2

Oxalsäure-bis-[3,5-bis-(2,3-dihydroxy-propyl-carbamoyl)-2,4,6-trijod-N-(2-hydroxyäthyl)-anilid]

a) In eine Lösung von 188 g (142 mMol) Oxalsäure-bis-[3,5-bis-(chlorcarbonyl)-2,4,6-trijod-anilid] (Dioxangehalt berücksichtigt) in 376 ml Dimethylacetamid wird bei 50-60 °C unter Rühren innerhalb von 30 Minuten eine Lösung von 117 g (1,28 Mol) 1-Aminopropandiol-(2,3) in 234 ml Dimethylacetamid getropft Anschließend wird 4 Stunden bei ca. 50 °C und über Nacht bei Raumtemperatur weitergerührt. Die Lösung wird mit 15 ml konzentrierter Salzsäure angesäuert und im Vakuum bei 50 °C eingedampft. Der Rückstand wird in 2 l Wasser eingerührt, über Nacht gerührt, der Niederschlag abgesaugt und mit Wasser gewaschen. Nach Trocknen bei 50 °C im Vakuum erhält man 156 g (75 % der Theorie) Oxalsäure-bis-[3,5-bis-(2,3-dihydroxy-propyl-carbamoyl)-2,4,6-trijod-anilid] ; Fp. : Keine Zersetzung bis 310 °C.

b) Hydroxyäthylierung :

Unter Erwärmen auf 50 °C werden 3 g (130 mMol) Natrium in 120 ml Methanol zum Methylat umgesetzt und 130 ml 1,2-Propylenglykol zugegeben. Dazu gibt man 44 g (30 mMol) Oxalsäure-bis [3,5-bis-(2,3-dihydroxy-propyl-carbamoyl)-2,4,6-trijod-anilid] und fügt zur rascjeren Lösung 300 ml Methanol zu. Nach 15 Minuten bei 50 °C ist die Lösung Klar. Dann wird das Methanol im Vakuum abdestilliert, wobei eine Suspension entsteht. Man gibt nun bei ca. 50 °C unter kräftigem Rühren 8 ml (120 mMol) Chloräthanol tropfenweise zu und rührt 5 Stunden bei 50 °C und über Nacht bei Raumtemperatur nach. Die trübe Lösung wird tropfenweise in 2 l Aceton eigerührt und der entstehende Niederschlag wird nach einer Stunde abgesaugt. Danach wird der Niederschlag 30 Minuten mit 500 ml Aceton ausgehürt, abgesaugt und bei 50 °C in Vakuum getrocknet. Das Rohprodukt (51,3 g) wird in 10 %iger wässriger Lösung über 500 ml.

Kationenaustauscher (IR 120) gegeben und mit Wasser eluiert. Das Eluat wird auf ca. 400 ml eingeengt und auf eine Säule mit ca. 500 ml Anionenaustauscher (IRA 410) gegeben und mit Wasser eluiert. Das Eluat wird mit 4 g Aktivkohle gerührt, abgesaugt und im Vakuum zur Trockne eingeengt. Zur Entfernung restlichen Propylenglykols und zur Gewinnung eines leichter handhabbaren Kristallisates wird der Rückstand ein- bis zweimal mit je 130 ml Äthanol ausgekocht, nach Abkühlen abgesaugt und im Vakuum bei 50 °C getrocknet. Man erhält 25 g (56 % der Theorie) Titelverbindung ; Fp. : 293-296 °C (Zersetzung).

### Beispiel 3

Oxalsäure-bis-[3,5-bis-(1,3-dihydroxy-isopropyl-carbamoyl)-2,4,6-trijod-N-(2-hydroxyäthyl)-anilid]

a) Analog Beispiel 2a) werden 199 g (150 mMol) Oxälsäure-bis-[3,5-bis-(chlorcarbonyl)-2,4,6-trijod-anilid] (Dioxangehalt berücksichtigt) mit 123 g (1,35 Mol) 2-Aminopropandiol-(1,3) (« Serinol ») umgesetzt und aufgearbeitet. Der Niederschlag wird 2 Tage lang mit Wasser gerührt, abgesaugt und getrocknet.

Man erhält 198 g (90 % der Theorie) Oxalsäure-bis-[3,5-bis-(1,3-dihydroxy-isopropylcarbamoyl)-2,4,6-trijod-anilid] ; Fp. : Keine Zersetzung bis 310 °C.

b) Hydroxyäthylierung :

Analog Beispiel 2b) wird die Natrium-Methylat/Propylenglykol-Lösung mit 44 g (30 mMol) Oxalsäure-bis-[3,5-bis-(1,3-dihydroxy-isopropyl-carbamoyl)-2,4,6-trijod-anilid] versetzt und Methanol zugegeben. Nach einstündigem Erhitzen auf 70 °C wird das Methanol im Vakuum abdestilliert und die erhaltene Suspension — wie im Beispiel 2b) beschrieben — mit 8 ml (120 mMol) 2-Chloräthanol versetzt und anschließend über Ionenaustauscher weiterverarbeitet.

Zur weiteren Reinigung wird das Rohprodukt (26 g) mit 110 ml Äthanol 16 Stunden unter Rückfluß gekocht. Nach Abkühlen wird abfiltriert und getrocknet. Man erhält 23 g (50 % der Theorie) Titelverbindung ; Fp. : Keine Zersetzung bis 310 °C.

Beispiel 4

Malonsäure-bis-[3,5-bis-(2,3-dihydroxy-propyl-carbamoyl)-2,4,6-trijod-N-(2-hydroxy-äthyl)-anilid]

a) Analog Beispiel 2a) werden 75 g (50 mMol) Malonsäure-bis-[3,5-bis-(chlorcarbonyl)-2,4,6-trijod-anilid] (Dioxangehalt berücksichtigt) mit 41 g (450 mMol) 1-Aminopropandiol umgesetzt und aufgearbeitet. Das Rohprodukt wird jedoch mit 700 ml Wasser 2 Tage verrühhrt, der Niederschlag abgesaugt, mit Wasser gewaschen und bei 50 °C im Vakuum getrocknet. Man erhält 59 g (80 % der Theorie) Malonsäure-bis-[3,5-bis-(2,3-dihydroxy-propyl-carbamoyl)-2,4,6-trijod-anilid] ; Fp. : 262-268 °C (Zersetzung).

b) Hydroxyäthylierung :

Analog Beispiel 2b) wird die aus 1,5 g (65 mMol) Natrium, Methanol und 1,2-Propandiol hergestellte Lösung mit 22,2 g (15 mMol) Malonsäure-bis-[3,5-bis-(2,3-dihydroxypropyl-carbamoyl)-2,4,6-trijod-anilid] versetzt und mit 150 ml Methanol verdünnt. Nach 2,5-stündigem Rühren bei ca. 60 °C ist die Substanz gelöst. Nach Abdestillieren des Methanols im Vakuum liefert die nun folgende Hydroxyäthylierung mit 4 ml (60 mMol) Chloräthanol nach der Aufarbeitung über Ionenaustauscher, Kochen mit 165 ml n-Butanol anstelle von Äthanol und Ausrühren mit Diäthyläther sowie nach Trocknen in Vakuum 15 g (64 % der Theorie) der Titelverbindung ; Fp. : 238 °C (Zersetzung).

Beispiel 5

Malonsäure-bis-[3,5-bis-(1,3-dihydroxy-isopropyl-carbamoyl)-2,4,6-trijod-N-(2-hydroxyäthyl)-anilid]

a) Analog Beispiel 2a) werden 252 g (192 mMol) Malonsäure-bis-[3,5-bis-(chlorcarbonyl)-2,4,6-trijod-anilid] (Dioxangehalt berücksichtigt) mit 164 g (1,8 Mol) 2-Aminopropandiol-(1,3) (« Serinol ») umgesetzt und aufgearbeitet. Das Rohprodukt wird jedoch 2 Tage mit nur 1 Liter Wasser verrührt. Man erhält 226 g (79,5 % der Theorie) Malonsäure-bis-[3,5-bis-(1,3-dihydroxy-isopropyl-carbamoyl)-2,4,6-trijod-anilid] ; Fp. : 289-298 °C (Zersetzung).

b) Hydroxyäthylierung :

Analog Beispiel 2b) wird die Natriummethylat/Propylenglykol-Lösung aus 15,6 g (680 mMol) Natrium mit 236,6 g (157 mMol) Malonsäure-bis-[3,5-bis-(1,3-dihydroxy-isopropyl-carbamoyl)-2,4,6-trijod-anilid] versetzt und mit 1 Liter Methanol verdünnt. Nach Lösen und Abdestillieren des Methanols im Vakuum werden 42 ml (628 mMol) 2-Chloräthanol zugegeben. Die übliche Aufarbeitung liefert nach dem Auskochen mit 800 ml Athanol 147 g (60 % der Theorie) der Titelverbindung ; Fp. 300 °C.

Beispiel 6

Oxalsäure-bis-[3,5-bis-(2,3-dihydroxy-propyl-N-methyl-carbamoyl)-2,4,6-trijod-N-(2-hydroxyäthyl)-anilid]

Analog Beispiel 2b) wird die Natriummethylat/1,2-Propylenglykol-Lösung (hergestellt aus 1,1 g (48 mMol) Natrium) mit der Lösung von 23,3 g (15 mMol) Oxalsäure-bis-[3,5-bis-(2,3-dihydroxy-propyl-N-methyl-carbamoyl)-2,4,6-trijod-anilid] versetzt und nach Abdestillieren des Methanols im Vakuum mit 3 ml (45 mMol) Chloräthanol umgesetzt. Nach der Behandlung mit Ionenaustauschern und Reinigung mit Aktiv-Kohle werden nach Eindampfen 17,4 g (72 % der Theorie) der Titelverbindung erhalten ; Fp. : 257-259 °C (Zersetzung).

Beispiel 7

3,6-Dioxakorksäure-bis-[3,5-bis-(1,3-dihydroxy-isopropyl-carbamoyl)-2,4,6-trijod-N-(2-hydroxy-äthyl)-anilid]

a) Analog Beispiel 2a) werden 34,1 g (24 mMol) 3,6-Dioxakorksäure-bis-[3,5-bis-(chlorcarbonyl)-2,4,6-trijod-anilid] (Dioxangehalt berücksichtigt), gelöst in 68 ml Dimethylacetamid, mit der Lösung von 19,9 g (219 mMol) 2-Aminopropandiol-(1,3) (« Serinol ») in 60 ml Dimethylacetamid umgesetzt. Nach dem Ansäuern und Einengen wird der Rückstand — gemäß Beispiel 1a) — mit Ionenaustauchern behandelt. Man erhält nach der Aufarbeitung 20,7 g (13,3 mMol) 3,6-Dioxakorksäure-bis-[3,5-bis-(1,3-dihydroxy-isopropyl-carbamoyl)-2,4,6-trijod-anilid] ; Fp. : Keine Zersetzung bis 310 °C.

b) Hydroxyäthylierung :

Analog Beispiel 2b) wird die Natriummethylat/1,2-Propylenglykol-Lösung (hergestellt aus 1,02 g (44,3 mMol) Natrium) mit 16,3 g (10,3 mMol) 3,6-Dioxakorksäure-bis-[3,5-bis-(1,3-dihydroxy-isopropyl-carbamoyl)-2,4,6-trijod-anilid] versetzt und mit 50 ml Methanol verdünnt. Nach Lösen und Abdestillieren des Methanols in Vakuum werden 2,8 ml (41,2 mMol) Chloräthanol zugegeben. Nach der Aufarbeitung und nach dem Auskochen mit 60 ml Äthanol erhält man 11,3 g (67 % der Theorie) der Titelverbindung : Fp. : 251-265 °C (Zersetzung).

Beispiel 8

Malonsäure-bis-[3,5-bis-(bis-2-hydroxy-äthylcarbamoyl)-2,4,6-trijod-N-(2-hydroxyäthyl)-anilid]

a) Analog Beispiel 2a) werden 131 g (100 mMol) Malonsäure-bis-[3,5-bis-(chlorcarbonyl)-2,4,6-trijod-anilid] (Dioxangehalt berücksichtigt) mit 94,5 g (900 mMol) Diäthanolamin umgesetzt und aufgearbeitet. Der Rückstand wird jedoch nur mit 1 Liter Wasser verrührt. Man erhält 114 g (75 % der Theorie) Malonsäure-bis-[3,5-bis-(bis-2-hydroxyäthylcarbamoyl)-2,4,6-trijod-anilid] ; Fp. : 235-244 °C (Zersetzung).

b) Hydroxyäthylierung :

Analog Beispiel 2b) wird die Natriummethylat (1,2-Propylenglykol-Lösung (hergestellt aus 4,94 g (215 mMol) Natrium) mit 76,7 g (50 mMol) Malonsäure-bis-[3,5-bis-bis-hydroxyäthylcarbamoyl)-2,4,6-trijod-anilid] versetzt und die Mischung mit Methanol verdünnt. Nach Lösen und Abdestillieren des Methanols im Vakuum werden 13,4 ml (200 mMol) 2-Chloräthanol zugegeben. Nach der Aufarbeitung und Entsalzung über Ionenaustauscher — gemäß Beispiel 2b) — wird das Rohprodukt durch 16-stündiges Kochen in 350 ml Äthanol zur Kristallisation gebracht. Man erhält 61,3 g (75,7 % der Theorie) der Titelverbindung ; Fp. 249-258 °C (Zersetzung).

Beispiel 9

Herstellung einer gebrauchsfertigen Lösung :

| | |
|---|---|
| Malonsäure-bis-[3,5-bis-(1,3-dihydroxy-isopropyl-carbamoyl)-2,4,6-trijod-N-(2-hydroxyäthyl)-anilid] | 617,08 g |
| Calciumdinatriumedetat | 0,10 g |
| Natriumbicarbonat | 1,08 g |
| Aqua bidestillata | ad 1 000 ml |

Die schattengebende Substanz wird nach Zusatz des Calciumdinatriumedetats in etwas Aqua bidestillata gelöst und der pH-Wert der Lösung wird durch Zugabe von Natrium-bicarbonat auf 7 gebracht. Nachdem man das Volumen durch Zugabe von Aqua bidestillata auf 1 000 ml gebracht hat, wird die Lösung anschließend hitzesterilisiert. Der Jodgehalt beträgt 300 mg/ml.

Beispiel 10

Malonsäure-bis-[3,5-bis-(2,3-dihydroxy-1-hydroxymethyl-propylcarbamoyl)-2,4,6-trijod-N-(2-hydroxyäthyl-anilid]

a) Analog Beispiel 1a) werden 131 g (100 mMol) Malonsäure-bis-[3,5-bis-(chlorcarbonyl)-2,4,6-trijod-anilid] (Dioxangehalt berücksichtigt) mit 72,5 g (450 mMol) 6-Amino-2,2-dimethyl-1,3-dioxepin-5-ol oder mit 55,4 g 1,2,4-Trihydroxy-3-amino-butan in Gegenwart von 107 ml (450 mMol) Tributylamin umgesetzt und aufgearbeitet. Man erhält 116 g (73 % der Theorie) Malonsäure-bis-[3,5-bis-(2,3-dihydroxy-1-hydroxymethyl-propylcarbamoyl)-2,4,6-trijod-anilid] ; Fp. : 254-263 °C (Zersetzung).

b) Hydroxyäthylierung :

Eine Lösung von Natriummethylat (hergestellt aus 4,94 g Natrium) in 200 ml Methanol und 215 ml 1,2-Propylenglykol wird bei 50 °C mit 79,9 g (50 mMol) Malonsäure-bis-[3,5-bis-(2,3-dihydroxy-1-hydroxymethylpropyl)-2,4,6-trijod-anilid] versetzt. Nach 15 Minuten wird die Lösung im Vakuum vom Methanol befreit, bei 50 °C unter kräftigem Rühren mit 13,4 ml (200 mMol) Chloräthanol versetzt, 5 Stunden bei dieser Temperatur gehalten und über Nacht ohne Heizung weitergerührt. Die übliche Aufarbeitung durch Fällen mit Aceton und Entsalzen über Ionenaustauscher liefert einen Eindampfrückstand, der durch

Kochen mit 350 ml Athanol kristallisiert. Ausbeute an Titelverbindung 55,5 g (61,2 % der Theorie) ; Fp. : 245-257 °C (Zersetzung)

Beispiel 11

Malonsäure-bis-[3,5-bis-(1,3-dihydroxy-isopropyl-carbamoyl)-2,4,6-trijod-N-(2,3-dihydroxypropyl)-anilid]

Eine Lösung von Natriummethylat (hergestellt aus 4,94 g Natrium) in 300 ml Methanol und 215 ml 1,2-Propylenglykol wird bei 50 °C mit 75,1 g (50 mMol) Malonsäure-bis-[3,5-bis-(1,3-dihydroxy-isopropyl-carbamoyl)-2,4,6-trijod-anilid] versetzt. Nach 15 Minuten wird die Lösung im Vakuum vom Methanol befreit, bei 50 °C unter kräftigem Rühren mit 16,7 ml (200 mMol) 3-Chlorpropandiol versetzt, 5 Stunden bei dieser Temperatur gehalten und über Nacht ohne Heizung weitergerührt. Die übliche Aufarbeitung durch Fällen mit Aceton und Entsalzen über lonenaustauscher gemäß Beispiel 2b), liefert einen Eindampfrückstand der durch Kochen mit 350 ml Isopropanol kristallisiert. Ausbeute an Titelverbindung 50,7 g (62,3 % der Theorie) ; Fp. : 229-242 °C (Zersetzung).

**Ansprüche**

1. N-Hydroxyalkylierte Dicarbonsäure-bis-(3,5-dicarbamoyl-2,4,6-trijod-anilide) der allgemeinen Formel I

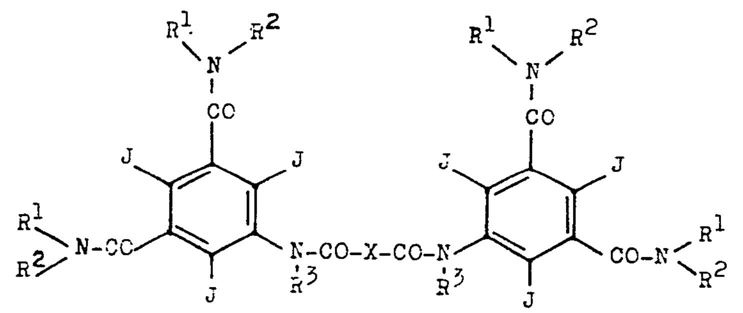

(I)

worin $R^1$ einen niederen gerad- oder verzweigtkettigen Mono- oder Polyhydroxyalkylrest mit 2 bis 8 Kohlenstoffatomen der durch 1 bis 5 Hydroxygruppen substituiert ist, darstellt, $R^2$ ein Wasserstoffatom, einen Alkylrest mit 1 bis 4 Kohlenstoffatomen oder $R^1$, $R^3$ einen Mono- oder Dihydroxyalkyl-rest mit 2 bis 3 Kohlenstoffatomen bedeutet und X eine Direkt bindung oder gegebenerfalls verzweigtkettiges Alkylen mit 1 bis 6 Kohlenstoffatomen, das durch 1 oder mehrere Sauerstoffatome unterbrochen oder durch Hydroxy- oder Alkoxygruppen substituiert sein kann, darstellt.

2. Malonsäure-bis-[3,5-bis-(1,3-di-hydroxy-isopropyl-carbamoyl)-2,4,6-trijod-N-(2-hydroxyäthyl)-anilid].

3. Malonsäure-bis-[3,5-bis-(2,3-dihydroxypropyl-N-methyl-carbamoyl)-2,4,6-trijod-N-(2-hydroxyäthyl)-anilid].

4. Malonsäure-bis-[3,5-bis-(2,3-dihydroxy-1-hydroxymethyl-propylcarbamoyl)-2,4,6-trijod-N-(2-hydroxy-äthyl)-anilid).

5. Oxalsäure-bis-[3,5-bis-(1,3-dihydroxy-isopropyl-carbamoyl)-2,4,6-trijod-N-(2-hydroxyäthyl)-anlid].

6. Oxalsäure-bis-[3,5-bis-(2,3-dihydroxy-propyl-N-methyl-carbamoyl)-2,4,6-trijod-N-(2-hydroxyäthyl)-anilid].

7. 3,6-Dioxakork-säure-bis-[3,5-bis-(1,3-dihydroxy-isopropyl-carbamoyl)-2,4,6-trijod-N-(2-hydroxy-äthyl)-anilid].

8. Malonsäure-bis-[3,5-bis-(1,3-dihydroxy-isopropyl-carbamoyl)-2,4,6-trijod-N-(2,3-dihydroxypropyl)-anilid].

9. Röntgenkontrastmittel, enthaltend mindestens eine Verbindung gemäß Anspruch 1 bis 8.

10. Verfahren zur Herstellung von N-hydroxy-alkylierten Dicarbonsäure-bis-(3,5-dicarbamoyl-2,4,6-trijod-aniliden) der allgemeinen Formel I, dadurch gekennzeichnet, daß man in an sich bekannter Weise ein Dicarbonsäure-bis-(3,5-dicarbamoyl-2,4,6-trijod-anilid) der allgemeinen Formel II

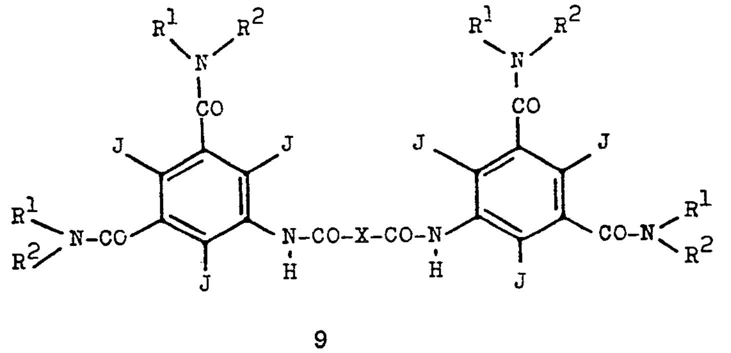

(II)

worin $R^1$, $R^2$ und X die obengenannte Bedeutung besitzt und im Molekül vorhandene freie Hydroxylgruppen in geschützter Form vorliegen können, mit einer Verbindung der allgemeinen Formel III

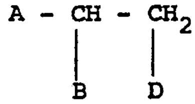

(III)

worin

A ein Wasserstoffatom oder eine $CH_2OH$-Gruppe darstellt und

B und D entweder gemeinsam das Sauerstoffatom eines Oxidoringes bedeuten oder

B eine Hydroxygruppe und

D ein Chlor- oder Bromatom oder eine Sulfat- oder Alkylsulfatgruppe darstellen,

N-hydroxy-alkyliert und gewünschtenfalls anschließend geschützte Hydroxylgruppen in Freiheit setzt.

**Claims**

1. N-hydroxyalkylated dicarboxylic acid bis-(3,5-dicarbamoyl-2,4,6-triiodoanilides) of the general formula I

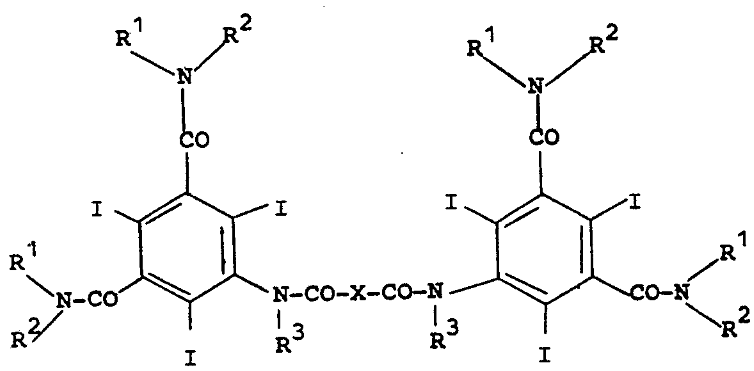

(I)

in which $R^1$ represents a lower straight-chain or branched-chain mono- or poly-hydroxyalkyl radical having from 2 to 8 carbon atoms which is substituted by from 1 to 5 hydroxy groups, $R^2$ represents a hydrogen atom, an alkyl radical having from 1 to 4 carbon atoms or $R^1$, $R^3$ represents a mono- or dihydroxyalkyl radical having 2 or 3 carbon atoms, and X represents a direct bond or an optionally branched-chain alkylene having from 1 to 6 carbon atoms which may be interrupted by 1 or more oxygen atoms or substituted by hydroxy or alkoxy groups.

2. Malonic acid bis-[3,5-bis-(1,3-dihydroxyisopropylcarbamoyl)-2,4,6-triiodo-N-(2-hydroxyethyl)-anilide].

3. Malonic acid bis-[3,5-bis-(2,3-dihydroxypropyl-N-methylcarbamoyl)-2,4,6-triiodo-N-(2-hydroxyethyl)-anilide].

4. Malonic acid bis-[3,5-bis-(2,3-dihydroxy-1-hydroxymethylpropylcarbamoyl)-2,4,6-triiodo-N-(2-hydroxyethyl)-anilide].

5. Oxalic acid bis-[3,5-bis-(1,3-dihydroxyisopropyl-carbamoyl)-2,4,6-triiodo-N-(2-hydroxyethyl)-anilide].

6. Oxalic acid bis-[3,5-bis-(2,3-dihydroxypropyl-N-methylcarbamoyl)-2,4,6-triiodo-N-(2-hydroxyethyl)-anilide].

7. 3,6-Dioxasuberic acid bis-[3,5-bis-(1,3-dihydroxyisopropylcarbamoyl)-2,4,6-triiodo-N-(2-hydroxyethyl)-anilide].

8. Malonic acid bis-[3,5-bis-(1,3-dihydroxyisopropylcarbamoyl)-2,4,6-triiodo-N-(2,3-dihydroxypropyl)-anilide].

9. X-ray contrast agents containing at least one compound according to claims 1 to 8.

10. Process for the manufacture of N-hydroxyalkylated dicarboxylic acid bis-(3,5-dicarbamoyl-2,4,6-triiodoanilides) of the general formula I, characterised in that in a manner known per se a dicarboxylic acid bis-(3,5-dicarbamoyl-2,4,6-triiodoanilide) of the general formula II

(See formula II, page 11)

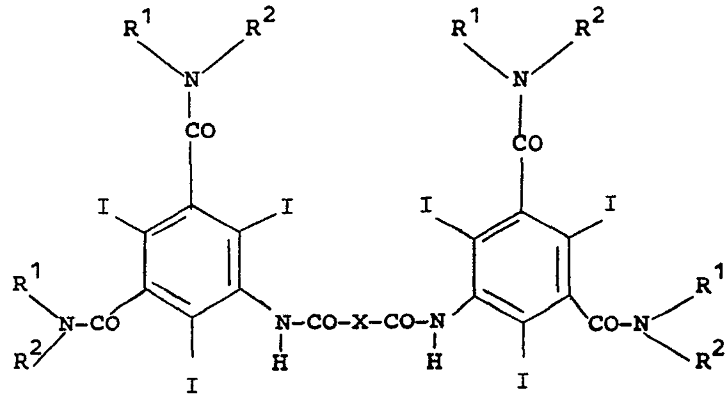

(II)

in which $R^1$, $R^2$ and X have the above-mentioned meanings and free hydroxy groups present in the molecule may be in protected form, is N-hydroxyalkylated with a compound of the general formula III

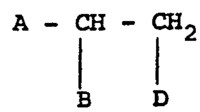

(III)

in which

A represents a hydrogen atom or a $CH_2OH$ group and

B and D either represent, together, the oxygen atom of an oxido ring, or

B represents a hydroxy group and

D represents a chlorine or bromine atom or a sulphate group or an alkyl sulphate group,

and if desired, protected hydroxy groups are then freed.

**Revendications**

1. Bis-(dicarbamoyl-3,5 tri-iodo-2,4,6 anilides) N-hydroxyalkylés d'acides dicarboxyliques qui répondent à la formule générale I

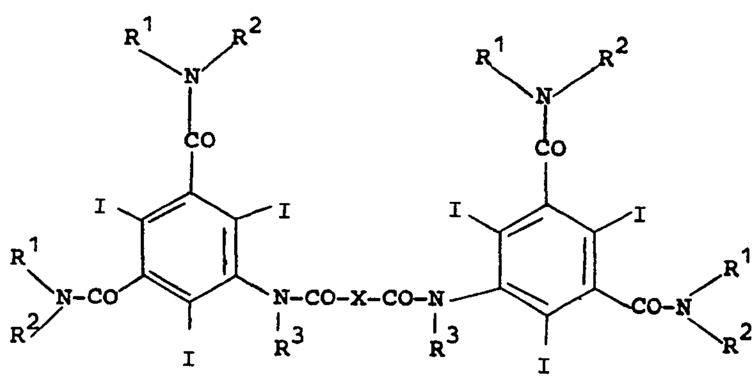

(I)

dans laquelle $R^1$ représente un radical monohydroxyalkyle ou polyhydroxyalkyle inférieur, linéaire ou ramifié, qui contient de 2 à 8 atomes de carbone et qui peut porter de 1 à 5 radicaux hydroxy, $R^2$ représente un atome d'hydrogène, un radical alkyle contenant de 1 à 4 atomes de carbone ou l'un des radicaux qui viennent d'être définis pour $R^1$, $R^3$ représente un radical monohydroxyalkyle ou dihydroxyalkyle contenant 2 ou 3 atomes de carbone et X représente une liaison directe ou un radical alkylène contenant de 1 à 6 atomes de carbone, éventuellement ramifié, qui peut être interrompu par un ou plusieurs atomes d'oxygène ou porter des radicaux hydroxy ou alcoxy.

2. Bis-[bis-(hydroxyméthyl-1 hydroxy-2 éthyl-carbamoyl)-3,5 tri-iodo-2,4,6 N-(hydroxy-2 éthyl)-anilide] de l'acide malonique.

3. Bis-[bis-(N-)dihydroxy-2,3 propyl)-N-méthyl-carbamoyl)-3,5 tri-iodo-2,4,6 N-(hydroxy-2 éthyl)-anilide] de l'acide malonique.

4. Bis-[bis-(dihydroxy-2,3 hydroxyméthyl-1 propyl-carbamoyl-3,5 tri-iodo-2,4,6 N-(hydroxy-2 éthyl)-anilide] de l'acide malonique.

5. Bis-[bis-(hydroxyméthyl-1 hydroxy-2 éthyl-carbamoyl)-3,5 tri-iodo-2,4,6 N-(hydroxy-2 éthyl)-anilide] de l'acide oxalique.

6. Bis-[bis-N-(dihydroxy-2,3 propyl)-N-méthyl-carbamoyl)-3,5 tri-iodo-2,4,6 N-(hydroxy-2 éthyl)-anilide] de l'acide oxalique.

7. Bis-[bis-(hydroxyméthyl-1 hydroxy-2 éthyl-carbamoyl)-3,5 tri-iodo-2,4,6 N-(hydroxy-2 éthyl)-anilide] de l'acide dioxa-3,6 subérique.

8. Bis-[bis-(hydroxyméthyl-1 hydroxy-2 éthyl-carbamoyl)-3,5 tri-iodo-2,4,6 N-(dihydroxy-2,3 propyl)-anilide] de l'acide malonique.

9. Agent de contraste pour rayons X qui contient au moins un composé selon l'une quelconque des revendications 1 à 8.

10. Procédé de préparation de bis-(dicarbamoyl-3,5 tri-iodo-2,4,6 anilides) N-hydroxyalkylés d'acides dicarboxyliques qui répondent à la formule générale I, procédé caractérisé en ce qu'on hydroxyalkyle à l'azote, de manière connue, un bis-(dicarbamoyl-3,5 tri-iodo-2,4,6 anilide) d'acide dicarboxylique répondant à la formule générale II :

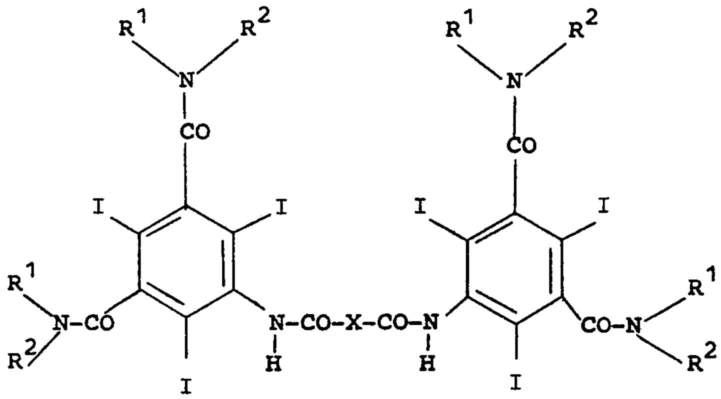

(II)

dans laquelle $R^1$, $R^2$ et X ont les significations précédemment données et les radicaux hydroxy libres éventuellement présents peuvent être sous une forme protégée, avec un composé répondant à la formule générale III :

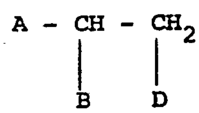

(III)

dans laquelle

A représente un atome d'hydrogène ou un radical —$CH_2OH$ et ou bien

B et D représentent ensemble l'atome d'oxygène d'un radical époxy, ou bien

B représente un radical hydroxy et

D représente un atome de chlore ou de brome ou un radical sulfato ou alkylsulfato,

puis, si on le désire, on libère les radicaux hydroxy protégés.